# EUROPEAN PATENT SPECIFICATION

(11) **EP 3 606 480 B1**
(45) Date of publication and mention of the grant of the patent: **27.01.2021**
(21) Application number: 18720700.6
(22) Date of filing: 04.04.2018
(51) Int. Cl.: A61F 5/44, A61F 5/445

(54) **DRAINABLE OSTOMY POUCH OUTLET**
ENTLEERBARER OSTOMIEBEUTEL
ORIFICE DE SORTIE DE POCHE DE STOMIE DRAINABLE

(30) Priority: 05.04.2017 US 201762481876 P
(43) Date of publication of application: 12.02.2020
(73) Proprietor: Hollister Incorporated, Libertyville, IL 60048 (US)
(72) Inventor: TORSTENSEN, Jan, Libertyville, IL 60048 (US); WEINBERG, Robert, J., Libertyville, IL 60048 (US); FRISKE, Timothy, A., Libertyville, IL 60048 (US)
(74) Representative: FRKelly
(86) International application number: PCT/US2018/026019
(87) International publication number: WO 2018/187427

(56) References cited:
- GB-A- 2 346 328
- US-A1- 2002 013 558
- US-A1- 2003 109 838
- US-A1- 2012 022 478
- US-A1- 2013 253 456

## Description

### BACKGROUND

The present disclosure generally relates to ostomy appliances, and more particularly to drainable ostomy pouches having closure systems.

Ostomy pouches for collecting body waste are used by patients who have had surgery such as a colostomy, ileostomy, or urostomy. Ostomy pouches typically include flat, opposing side walls secured together along their edges to define a collection cavity. One of the side walls is provided with an opening to receive a stoma, and means to secure the pouch to the user, such as an adhesive barrier, so that body waste discharged through the stoma is received within the cavity.

The ostomy pouch may be a closed-end pouch for a single use, in which case the entire pouch is discarded after it has been substantially filled with stomal discharge. Alternatively, the ostomy pouch may be a drainable pouch with a discharge opening at its lower end, which may be closed during collection of body waste material but may be opened for draining body waste material from the pouch after a period of use. Such drainable pouches are disclosed, for example, in Nolan, U.S. Patent No. 3,523,534, and Jensen et al., U.S. Patent No. 4,411,659.

The discharge opening of drainable pouches is typically defined at the end of a narrowed outlet portion, which is provided with closure means for maintaining the discharge opening in a sealed condition until waste material is to be drained from the pouch. The closure means may take the form of a clamp, as in the aforementioned Nolan patent, or a device such as conventional wire ties or wraps for securing the outlet portion in an upwardly-rolled condition.

For quality of life of the users, drainable pouches should be easy to drain without risking soiling of clothes or the surroundings. They also should be easy to close securely after being drained and amenable to being cleaned after drainage and before closing again, such that the risk of unpleasant odor is substantially reduced. Most importantly, the closure means should provide a secure seal when closed to minimize the risk of leakage.

Many different solutions concerning the closing, cleaning and drainage operations have been proposed and implemented. For example, Villefrance et al., U.S. Patent No. 7,879,015 and Friske et al., U.S. Patent No. 8,672,907, disclose drainable pouches having integral closure systems. US2002013558A1 discloses an ostomy pouch for both one and two-piece ostomy appliances having an integral closure at the bottom of the pouch to allow the pouch to be emptied and resealed without the use of a separate closure. The integral closure has mating male and female parts that are locked together either manually by finger pressure or by a sliding mechanism. The integral closure provides the ostomy appliance with a leak-proof, odor-proof seal when in a closed, sealed, and locked position. A slider, when the appliance is dosed, can be moved along the integral closure, thereby separating the mated male and female parts to provide an opening through which the stored contents within the ostomy pouch can be emptied. After the stored contents have been emptied, the slider can reseal the pouch by rejoining the male and female parts. For obvious reasons, further improvements in the closure systems for easier operation and reduced risk of leakage are much desired by users.

Accordingly, there is a need for an improved closure system for drainable pouches.

### BRIEF SUMMARY

The invention is defined in the claims.

A drainable ostomy pouch comprising an outlet device including a closure member and a slider is provided according to various embodiments. The outlet device may be configured to provide an improved drainable pouch outlet that is easier to handle during draining and cleaning compared to conventional drainable pouch outlets. The outlet device may provide a liquid tight closure via a simple sliding motion using a slider that is configured to engage with a closure member. The outlet device may also be configured to allow a user to open the pouch outlet by applying pressure along opposite sides of the outlet device to provide a discharge opening having angled corners. Further, the outlet device may be formed from a relatively rigid material and provided with bend lines to facilitate opening of the pouch outlet. Such features may eliminate difficulties in opening an ostomy pouch due to the pouch outlet walls sticking together and bending in the same direction. Further, by providing a discharge opening having angled corners, the outlet device may provide improved flow control of body waste draining out through the discharge opening.

In one aspect, a drainable ostomy pouch may include a bodyside wall and an outer wall defining a cavity therebetween for collecting stomal discharge, and a downwardly extending outlet portion terminating in an outlet opening for draining stomal discharge contents collected in the cavity. The drainable ostomy pouch may also include an outlet device comprising a closure member and a slider attached to the outlet portion. The closure member may include a body including a first opening, a second opening, and four bend zones defined therebetween, and at least one bar arranged on the body proximate the second opening. The closure member may be configured to bend along the four bend zones to provide a discharge opening having four angled corners in an open position and to be pressed flat in a closed position. The slider may be configured to slide over the at least one bar and engage with the closure member to close the discharge opening.

The four bend zones may extend longitudinally along the body between the first and second openings. In an embodiment, the body may be formed from a single piece of a polymeric material, in which the bend zones are defined by crease lines or perforated lines. Alternatively, the body may be formed from four pieces of a polymeric sheet material, in which each piece defines a panel of the body, and four boundaries between the panels define the four bend zones.

In some embodiment, the bend zones may be symmetrically arranged, such that a distance between adjacent bend zones is generally equal to each other, and the closure member provides a generally square-shaped discharge opening in a fully opened position. In another embodiment, the bend zones may include first, second, third and fourth bend zones, wherein the first bend zone is adjacent the second and fourth bend zones. A distance between the first and second bend zone may be generally equal to a distance between the third and fourth bend zones, and a distance between the second and third bend zones may be generally equal to a distance between the first and fourth bend zones. In such an embodiment, the distance between the first and second bend zones may be greater than the distance between the second and third bend zones, such that the closure member may provide a generally rectangular discharge opening in a fully opened position.

In an embodiment, the at least one bar may include four bars, in which each of the four bars is arrange on an outer surface of the body proximate the second opening, such that each of the four bend zones is arranged between adjacent bars.

The closure member may include a soft material arranged proximate the second opening. The soft material may be configured and arranged to be compressed together when the closure member engages with the slider to provide a liquid tight seal closure. In an embodiment, the soft material may be attached to inner surfaces of the body opposite the at least one bar. In another embodiment, the soft material may be arranged on outer surfaces of the body between the at least one bar and the body. The soft material may be formed from a foam material.

In some embodiments, the closure member may be arranged in the outlet portion, such that the at least one bar extends beyond the outlet opening, and outer surfaces of the body are attached to adjacent inner surfaces of the outlet portion. The outlet device may be configured and arranged to allow a user to press along sides of the outlet portion to provide the discharge opening having the four angled corners.

In any of the forgoing embodiments, the drainable ostomy pouch may include a two-part fastening system comprising first and second fastener strips configured for securing the outlet portion in a folded up closed position. The outlet portion may be configured to be folded up two times to engage the first and second fastener strips to secure the folded outlet portion, wherein the outlet portion may be configured to fold along an upper periphery of the closure member proximate the first opening for a first fold and fold along a lower periphery of the closure member proximate the second opening for the second fold.

In an embodiment, the slider may have a generally u-shaped body including a channel configured to receive a peripheral portion of the closure member proximate the second opening including the at least one bar. In such an embodiment, the outlet device may be configured to provide a liquid tight seal when the slider engages the closure member and applies pressure over the peripheral portion of the closure member. Further, the slider may be configured to fold up longitudinally and slide over a side periphery of the outlet portion in an open position.

Other aspects, objectives and advantages will become more apparent from the following detailed description when taken in conjunction with the accompanying drawings.

### BRIEF DESCRIPTION OF THE DRAWINGS

The benefits and advantages of the present embodiments will become more readily apparent to those of ordinary skill in the relevant art after reviewing the following detailed description and accompanying drawings, wherein:
FIG. 1 is a perspective view of a drainable ostomy pouch including an outlet device comprising a closure member and a slider according to an embodiment;
FIG. 2 is a perspective view of the drainable ostomy pouch of FIG. 1 in an open position with the slider folded and held in place along a side periphery of a pouch outlet portion;
FIG. 3 is a perspective view of the drainable ostomy pouch of FIGS. 1 and 2 showing the outlet portion fully opened;
FIG. 4 is a perspective view of the outlet device of FIG. 1 including a closure member and a slider according to an embodiment;
FIG. 5 is a perspective view of the closure member of FIG. 4 in an open position;
FIG. 6 is a cross sectional side view of an outlet device including a closure member and a slider according to another embodiment;
FIG. 7 is a perspective view of the drainable pouch of FIG. 1 in a closed position;
FIG. 8 is a perspective view of the drainable pouch of FIG. 7 folded up once;
FIG. 9 is a perspective view of the drainable pouch of FIG. 7 folded up two times and secured in place with a pair of fasteners;
FIG. 10 is a schematic bottom view of a closure member according to an embodiment in a closed position; and
FIG. 11 is a schematic bottom view of the closure member of FIG. 10 in an open position.

### DETAILED DESCRIPTION

While the present disclosure is susceptible of embodiment in various forms, there is shown in the drawings and will hereinafter be described presently preferred embodiments with the understanding that the present disclosure is to be considered an exemplification and is not intended to limit the disclosure to the specific embodiments illustrated. The words "a" or "an" are to be taken to include both the singular and the plural. Conversely, any reference to plural items shall, where appropriate, include the singular.

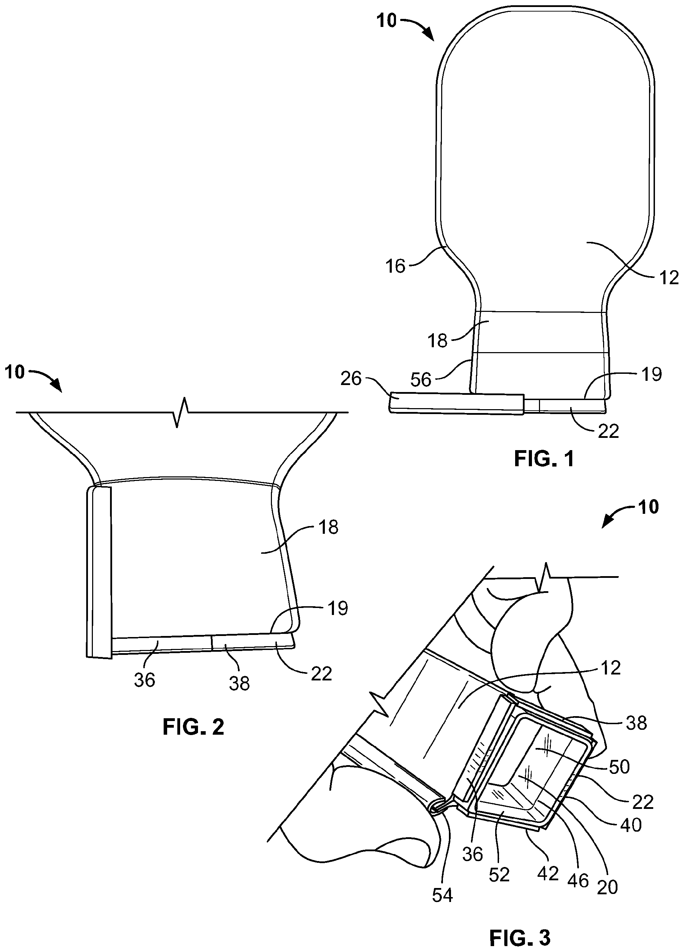

Referring now to the figures, FIGS. 1-3 show a drainable ostomy pouch 10 according to an embodiment. The drainable ostomy pouch 10 may include an outer wall 12 and a body-side wall 14, which may be joined along their peripheral edges 16 to define a cavity therebetween for collecting stomal discharge. The drainable ostomy pouch 10 may include a downwardly extending outlet portion 18 terminating in a discharge opening 20 for draining the contents collected in the cavity after a period of use. An outlet device 22 may be provided in the outlet portion 18 to facilitate opening of the discharge opening 20 and to provide liquid tight closure of the drainable ostomy pouch 10.

The pouch walls 12, 14 may be formed from a suitable flexible material, such as a polymeric film, which may be a monolayer or multilayer film. Each of the pouch walls 12, 14 may be formed of one continuous flexible film to define the entire pouch including the outlet portion 18. Alternatively, the walls of the outlet portion 18 may be formed of separate flexible films than the walls of the pouch body. That is, the walls of the outlet portion 18 may be formed from a different polymeric film than the walls of the pouch body.

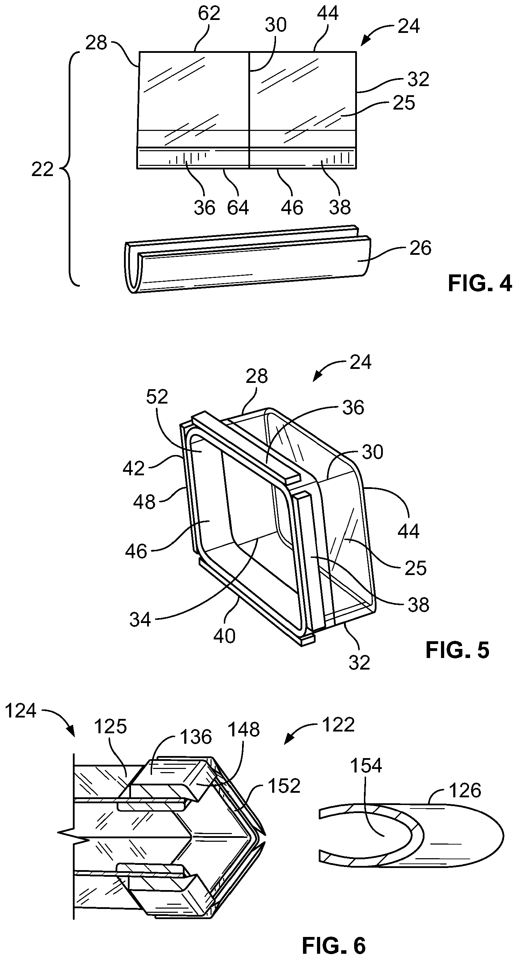

FIGS. 4-5 illustrate an embodiment of the outlet device 22 generally comprising a closure member 24 and a slider 26. The closure member 24 may include a generally rectangular shaped body 25 comprising a first opening 44, a second opening 46, and four bend zones 28, 30, 32, 34 defined therein. The four bend zones 28, 30, 32, 34 may extend longitudinally along the body 25 between the first and second openings 44, 46. The closure member 24 may also include bars 36, 38, 40, 42 arranged on outer surfaces of the body 25 proximate the second opening 46. FIG. 4 shows the closure member 24 in a closed position, in which the closure member 24 is pressed flat. FIG. 5 shows the closure member 24 in an open position, in which the closure member 24 is bent along four bend zones 28, 30, 32, 34 to provide the first and second openings 44, 46 having four angled corners defined at the four bend zones 28, 30, 32, 34.

The body 25 may be formed from a suitable material. In some embodiments, the body 25 may be formed from a relatively rigid polymeric material having a thickness of about 2 mil to about 40 mil. The body 25 may be formed from a single piece of polymeric material, wherein the bend zones 28, 30, 32, 34 may be defined by crease lines or perforated lines. Alternatively, the body 25 may be formed using four separate panels joined together, in which boundaries between the panels define the bend zones 28, 30, 32, 34.

In the embodiments of FIGS. 1-5, the outlet device 22 may include four bars 36, 38, 40, 42. Each of the four bars 36, 38, 40, 42 may be arranged proximate the second opening 46 between bend zones 28, 30, 32 34, such that each of the bend zones 28, 30, 32, 34 is arranged between adjacent bars. In another embodiment, the outlet device 22 may include two bars, each including a bend zone defined therein. In such an embodiment, the two bars may be arranged on opposite outer surfaces of the body 25 proximate the second opening 46, such that two of the bend zones defined in opposite sides of the body 25 are arranged between the two bars and other two bend zones defined in the body 25 line up with the bend zones defined in the bars. In yet another embodiment, the outlet device 22 may include a single bar configured to fit over the body 25 proximate the second opening 46 and including four bend zones defined therein. In such an embodiment, the bar may be arranged, such that the four bend zones defined in the body 25 and the four bend zones defined in the bar may align with each other to allow the outlet device to bend along the bend zones.

The bars 36, 28, 40, 42 may be formed from the same material as the body 25 or a different material. In an embodiment, the bars 36, 38, 40, 42 may be formed from a relatively rigid polymeric material having a greater thickness than the body 25. For example, the bars 36, 38, 40, 42 may be formed from a polymeric material having a thickness of about 5 mil to about 80 mil. In one embodiment, the closure member 24 including the body 25 and the bars 36, 38, 40, 42 may be formed via injection molding.

In an embodiment, the bend zones 28, 30, 32, 34 may be symmetrically arranged, such that a distance between adjacent bend zones is generally equal to each other. In such an embodiment, the closure member 24 may provide a generally square-shaped discharge opening 20 when fully opened as shown in FIG. 3. Each of the bars 36, 38, 40, 42 may be configured to have a generally rectangular shape with generally same width, length, and thickness. The bars 36, 38, 40, 42 may be provided in the areas between the four bend zones 28, 30, 32, 34 of the body 25 proximate the second opening 46, as shown in FIGS. 3 and 5.

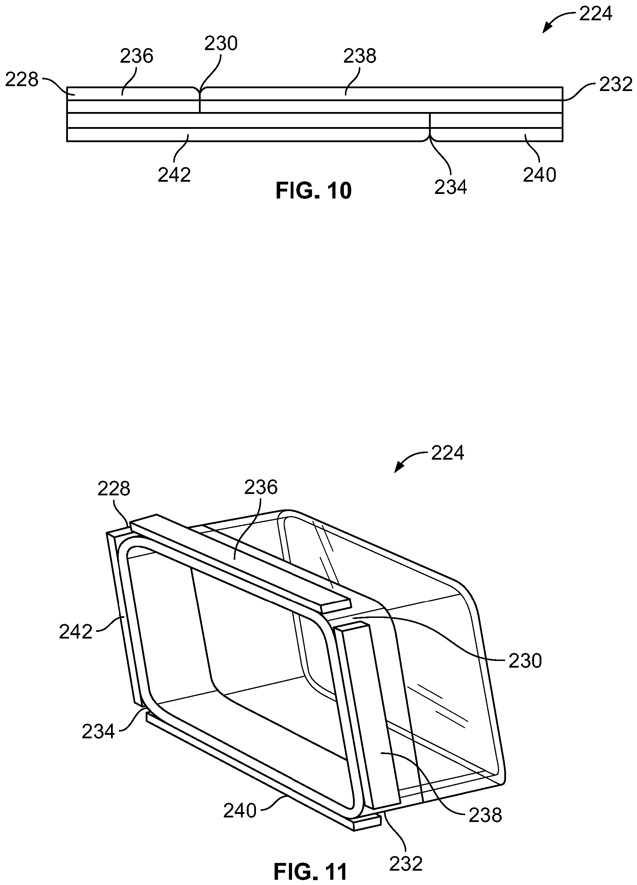

Alternatively, a closure member may be configured such that distances between adjacent bend zones are not all equal to each other. FIGS. 10-11 are schematic bottom views of a closure member 224 having such configuration of bend zones according to an embodiment. The closure member 224 may be configured similar to the closure member 24 of FIGS. 1-5 including a generally rectangular shaped body comprising a first opening, a second opening 246, four bend zones 228, 230, 232, 234, and four bars 236, 238, 240, 242. In this embodiment, a distance between the bend zones 228 and 230 is generally equal to a distance between the bend zones 232 and 234, and greater than a distance between the bend zones 230, 232 and a distance between the bend zones 228, 234. The distance between the bend zones 230, 232 and the distance between the bend zones 228, 234 are generally equal to each other.

As such, two longer bars 236, 240 and two shorter bars 238, 242 may be arranged between the bend zones 228, 230, 232, 234 as shown in FIG. 10. When opened, the closure member 224 may provide a generally rectangular discharge opening as shown in FIG. 11. The closure member 224 having such a configuration may be less likely to fold in on itself when compared to a closure member having bend zones arranged generally an equal distance from each other, such as the closure member 24 of FIGS. 1-5, to allow the closure member 224 to remain in an open position.

In an embodiment, each of the bars 36, 38, 40, 42 may be provided with a sharp edge 48 along a peripheral edge. The sharp edges 48 may be defined by a reduced thickness portion of the bars 36, 38, 40, 42 or a separate member having a sharper edge than the edge of the bars 36, 38, 40, 42. In such an embodiment, each of the bars 36, 38, 40, 42 may be arranged on the outer surfaces of the body 25 proximate the second opening 46, such that the sharp edges 48 may extend beyond an outer periphery of the body 25. The sharp edges 48 may be configured to reduce or prevent dripping and/or spilling when emptying body waste collected in an ostomy pouch through the discharge opening 20. In some embodiments, the bars 36, 38, 40, 42 may be configured to facilitate attachment of an adaptor for a night drainage bag.

The closure member 24 may also include a soft material 52 proximate the second opening 46 to facilitate sealing of the drainable ostomy pouch 10 in a closed position. In an embodiment, the soft material 52 may be attached to inner surfaces of the body 25 opposite the bars 36, 38, 40, 42 proximate the second opening 46 as shown in FIGS. 3 and 5. In another embodiment, the soft material may be arranged on outer surfaces of the body 25 between the bars 36, 38, 40, 42 and the body 25. The soft material 52 may be formed from a suitable soft material, such as a foam material, soft silicone, nitrile rubber or similar materials known for good sealing properties. For example, the soft material 52 may be formed from a closed cell foam material and arranged on the inner surfaces of the body 25 opposite the bars 36, 38, 40, 42. The soft material 52 may be configured and arranged to be compressed together when the closure member 24 engages with the slider 26 to provide a liquid tight seal closure.

FIG. 6 is a cross sectional side view of an outlet device 122 according to another embodiment. The outlet device 122 may be configured similar to the outlet device 22 and generally include a closure member 124 and a slider 126. The closure member 124 may include a body 125, bars 136, and a soft material 152. In this embodiment, each of the bars 136 may be provided with a protruding end portion 148 forming a sharp edge. The protruding end portions 148 may be formed from a suitable thin material having a thickness of about 2 mil to about 10 mil to provide the sharp edge extending from the bars 136.

The slider 26 may be configured to engage with the closure member 24 to provide a liquid tight closure for the ostomy pouch 10. In an embodiment, the slider 26 may be formed from a suitable polymeric material and configured to slide over the bars 36, 38, 40, 42 as shown in FIG. 1. In the embodiments of FIGS. 1-4 and 6, the slider 26, 126, may have a generally u-shaped body including a channel 54, 154 configured to receive a peripheral portion of the closure member 24, 124 including the bars 36, 38, 40, 42, 136. The outlet device 22, 124 may be configured to provide a liquid tight seal when the slider 26, 126 engages the closure member 24, 124 and applies pressure over the peripheral portion of the closure member 24, 124.

In an embodiment, the slider 26 may be configured to fold up longitudinally and slide over a side periphery 56 of the pouch outlet portion 18 as shown in FIGS. 2 and 3. In such an embodiment, the outlet device 22 may be provided with a stop (not shown) proximate a corner of the outlet device configured to prevent the slider 26 from sliding completed off the closure member 24. In an open position, the slider 26 may be folded up longitudinally and held in place along the side periphery 56 as shown in FIG. 2, which can prevent the slider 26 from being misplaced during draining and cleaning.

In an embodiment, the closure member 24 may be arranged in the outlet portion 18 of the drainable pouch 10, such that the bars 36, 38, 40, 42 extend beyond an outer periphery 19 of the outlet portion 18 as shown in FIGS. 1-3. In such an embodiment, outer surfaces of the body 25 may be attached to adjacent inner surfaces of the outlet portion 18 via heat sealing, an adhesive, or other similar known methods. In another embodiment, the closure member 24 may be arranged over the outlet portion 18, such that the inner surfaces of the body 25 are attached to the adjacent outer surfaces of the outlet portion 18.

When attached to the outlet portion 18, the outlet device 22 may allow a user to press along sides of the outlet portion 18 to provide the discharge opening 20 having angled corners defined at the four bend zones 28, 30, 32, 34. In some embodiments, the outlet device 22 may provide generally v-shaped corners in an open position as shown in FIGS. 3 and 5. Further, the outlet device may provide a generally square shaped discharge opening 20 in a fully opened position as shown in FIG.3. An outlet path 50 is defined in the closure member 24 between the first and second opening 44, 46 providing a flow path for draining body waste collected in the drainable pouch 10.

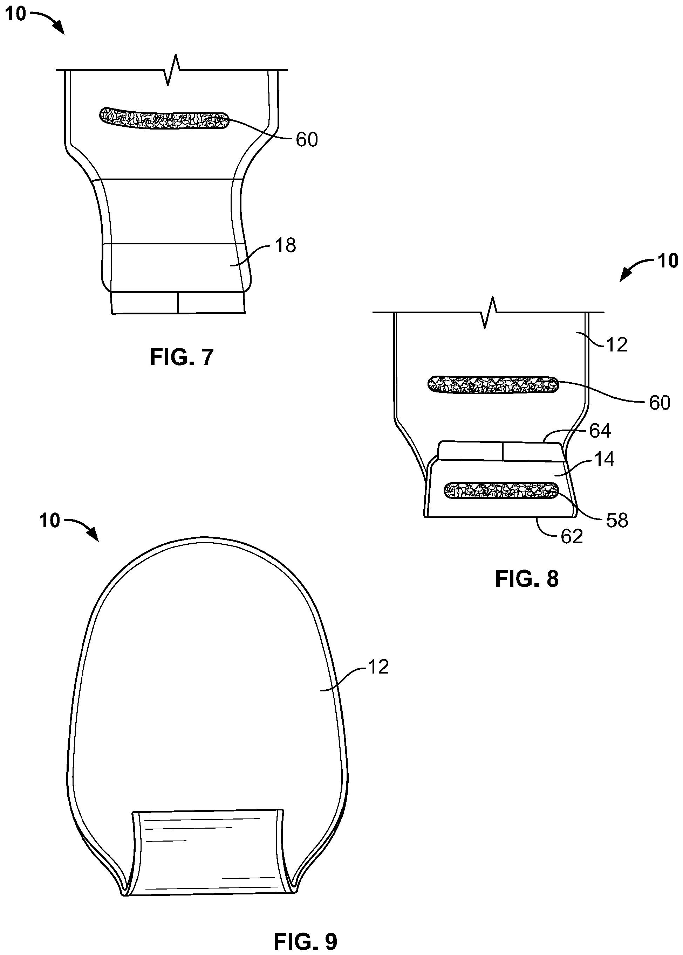

In an embodiment, the drainable pouch 10 may include a two-part fastening system comprising first and second fastener strips 58, 60 for securing the folded-up outlet portion 18 in a closed position as shown in FIGS. 7-9. The first fastener strip 58 may be provided on an outer surface of the body-side wall 14 in the outlet portion 18, while the second fastener strip 60 may be arranged on an outer surface of the outer wall 12 in a pouch portion as shown in FIGS. 7-8. The first and second fastener strips 58, 60 may include fastener elements, which are adapted for releasable interlocking engagement. In the embodiments of FIGS. 7-9, the drainable pouch 10 may be configured such that the outlet portion 18 may be folded up two times to engage the first and second fastener strips 58, 60 to secure the folded outlet portion 18. In this embodiment, the outlet portion 18 may be configured to fold along an upper periphery 62 of the closure member 24 (FIGS. 4 and 8) for a first fold and fold along a lower periphery 64 of the closure member 24 (FIGS. 8 and 9) for a second fold. The fastener strips 58, 60 may be formed from known fastening means, such as, for example, hook and loop fasteners as marketed under the Velcro trademark or pressure sensitive adhesive coatings.

From the foregoing it will be observed that numerous modifications and variations can be effectuated without departing from the scope of the novel concepts of the present disclosure. It is to be understood that no limitation with respect to the specific embodiments illustrated is intended or should be inferred.

## Claims

1. A drainable ostomy pouch (10), comprising:
a bodyside wall (14) and an outer wall (12)defining a cavity therebetween for collecting stomal discharge;
a downwardly extending outlet portion (18) terminating in an outlet opening (20) for draining stomal discharge contents collected in the cavity; and
an outlet device (22) attached to the outlet portion (18), wherein the outlet device (22) comprises a closure member (24) and a slider (26), **characterised in that** the closure member (24) comprises:
a body (25)including first opening (44), second opening (46), and four bend zones (28, 30, 32, 34) defined therebetween; and
at least one bar (36, 38, 40, 42) arranged on the body proximate the second opening (46);
wherein the closure member (24) is configured to bend along the four bend zones (28, 30, 32, 34) to provide a discharge opening (44, 46) having four angled corners in an open position and to be pressed flat in a closed position;
wherein the slider (26) is configured slide over the at least one bar (36, 38, 40, 42) and engage with the closure member (24) to close the discharge opening.

2. The drainable ostomy pouch (10) of claim 1, wherein the four bend zones (28, 30, 32, 34) extend longitudinally along the body between the first and second openings (44, 46).

3. The drainable ostomy pouch (10) of claim 1 or 2, wherein the body (25) is formed from a single piece of a polymeric material, wherein the bend zones (28, 30, 32, 34) are defined by crease lines or perforated lines.

4. The drainable ostomy pouch (10) of claim 1 or 2, wherein the body (25) is formed from four pieces of a polymeric sheet material, wherein each piece defines a panel of the body (25), wherein four boundaries between the panels define the four bend zones (28, 30, 32, 34).

5. The drainable ostomy pouch of any of claims 1-4, wherein the bend zones (28, 30, 32, 34) are symmetrically arranged, wherein a distance between adjacent bend zones is generally equal to each other, and wherein the closure member (24) provides a generally square-shaped discharge opening in a fully opened position.

6. The drainable ostomy pouch (10) of any of claims 1-4, wherein the bend zones (228, 230, 232, 234) include first (228), second (230), third (232) and fourth (234) bend zones, wherein the first bend zone is adjacent the second and fourth bend zones, wherein a distance between the first and second bend zone is generally equal to a distance between the third and fourth bend zones, and a distance between the second and third bend zones is generally equal to a distance between the first and fourth bend zones, wherein the distance between the first and second bend zones is greater than the distance between the second and third bend zones, and wherein the closure member (224) provides a generally rectangular discharge opening in a fully opened position.

7. The drainable ostomy pouch (10) of any of claims 1-6, wherein the at least one bar (36, 38, 40, 42) includes four bars (36, 38, 40, 42), wherein each of the four bars is arranged on an outer surface of the body (25) proximate the second opening (46), such that each of the four bend zones (28, 30, 32, 34) is arranged between adjacent bars.

8. The drainable ostomy pouch (10) of any of claims 1-7, wherein the closure member (24) includes a soft material (52) arranged proximate the second opening (46), wherein the soft material (52) is configured and arranged to be compressed together when the closure member (24) engages with the slider (26) to provide a liquid tight seal closure.

9. The drainable ostomy pouch (10) of claim 8, wherein the soft material (52) is attached to inner surfaces of the body (25) opposite the at least one bar (36, 38, 40, 42).

10. The drainable ostomy pouch (10) of claim 8, wherein the soft material (52) is arranged on outer surfaces of the body (25) between the at least one bar (36, 38, 40, 42) and the body (25).

11. The drainable ostomy pouch (10) of any of claims 8-10, wherein the soft material (52) is formed from a foam material.

12. The drainable ostomy pouch (10) of any of claims 1-11, wherein the closure member (24) is arranged in the outlet portion (18), such that the at least one bar (36, 38, 40, 42) extends beyond the outlet opening, wherein outer surfaces of the body (25) are attached to adjacent inner surfaces of the outlet portion (18), wherein the outlet device (22) is configured and arranged to allow a user to press along sides of the outlet portion (18) to provide the discharge opening having the four angled corners.

13. The drainable ostomy pouch (10) of any of claims 1-12, wherein the drainable ostomy pouch includes a two-part fastening system comprising first and second fastener strips (58, 60) configured for securing the outlet portion (18) in a folded up closed position.

14. The drainable ostomy pouch (10) of claim 13, wherein the outlet portion (18) is configured to be folded up two times to engage the first and second fastener strips (58, 60) to secure the folded outlet portion (18), wherein the outlet portion (18) is configured to fold along an upper periphery (62) of the closure member proximate the first opening (44) for a first fold and fold along a lower periphery (64) of the closure member proximate the second opening (46) for the second fold.

15. The drainable ostomy pouch (10) of any of claims 1-14, wherein the slider (26) has a generally u-shaped body including a channel (54) configured to receive a peripheral portion of the closure member (24) proximate the second opening including the at least one bar (36, 38, 40, 42), wherein the outlet device (22) is configured to provide a liquid tight seal when the slider (26) engages the closure member (24) and applies pressure over the peripheral portion of the closure member.

16. The drainable ostomy pouch (10) of any of claims 1-15, wherein the slider (26) is configured to fold up longitudinally and slide over a side periphery of the outlet portion (18) in an open position.

## Patentansprüche

1. Entleerbarer Ostomiebeutel (10), umfassend:
eine körperseitige Wand (14) und eine Außenwand (12), die einen Hohlraum dazwischen zum Sammeln von Stomaausscheidungen definieren;
einen sich nach unten erstreckenden Auslassabschnitt (18), der in einer Auslassöffnung (20) endet, um den in dem Hohlraum gesammelten Stomaausscheidungsinhalt zu entleeren; und
eine Auslassvorrichtung (22), die an dem Auslassabschnitt (18) angebracht ist, wobei die Auslassvorrichtung (22) ein Verschlusselement (24) und einen Schieber (26) umfasst, **dadurch gekennzeichnet, dass** das Verschlusselement (24) Folgendes umfasst:
einen Körper (25), der eine erste Öffnung (44), eine zweite Öffnung (46) und vier dazwischen definierte Biegezonen (28, 30, 32, 34) beinhaltet; und
mindestens eine Stange (36, 38, 40, 42), die an dem Körper nahe der zweiten Öffnung (46) angeordnet ist;
wobei das Verschlusselement (24) dazu konfiguriert ist, sich entlang der vier Biegezonen (28, 30, 32, 34) zu biegen, um eine Auslassöffnung (44, 46) bereitzustellen, die in einer offenen Position vier abgewinkelte Ecken aufweist und in einer geschlossen Position flach gedrückt werden kann;
wobei der Schieber (26) dazu konfiguriert ist, über die mindestens eine Stange (36, 38, 40, 42) zu gleiten und mit dem Verschlusselement (24) in Eingriff zu treten, um die Auslassöffnung zu schließen.

2. Entleerbarer Ostomiebeutel (10) nach Anspruch 1, wobei sich die vier Biegezonen (28, 30, 32, 34) in Längsrichtung entlang des Körpers zwischen der ersten und der zweiten Öffnung (44, 46) erstrecken.

3. Entleerbarer Ostomiebeutel (10) nach Anspruch 1 oder 2, wobei der Körper (25) aus einem Stück eines Polymermaterials gebildet ist, wobei die Biegezonen (28, 30, 32, 34) durch Falzlinien oder perforierte Linien definiert sind.

4. Entleerbarer Ostomiebeutel (10) nach Anspruch 1 oder 2, wobei der Körper (25) aus vier Stücken eines polymeren Folienmaterials gebildet ist, wobei jedes Stück eine Platte des Körpers (25) definiert, wobei vier Grenzen zwischen den Platten die vier Biegezonen (28, 30, 32, 34) definieren.

5. Entleerbarer Ostomiebeutel nach einem der Ansprüche 1-4, wobei die Biegezonen (28, 30, 32, 34) symmetrisch angeordnet sind, wobei ein Abstand zwischen benachbarten Biegezonen einander im Wesentlichen gleich ist, und wobei das Verschlusselement (24) in einer vollständig geöffneten Position eine im Allgemeinen quadratische Auslassöffnung bereitstellt.

6. Entleerbarer Ostomiebeutel (10) nach einem der Ansprüche 1-4, wobei die Biegezonen (228, 230, 232, 234) eine erste (228), eine zweite (230), eine dritte (232) und eine vierte (234) Biegezone beinhalten, wobei die erste Biegezone der zweiten und vierten Biegezone benachbart ist, wobei ein Abstand zwischen der ersten und der zweiten Biegezone im Allgemeinen gleich einem Abstand zwischen der dritten und der vierten Biegezone ist und ein Abstand zwischen der zweiten und der dritten Biegezone im Allgemeinen gleich einem Abstand zwischen der ersten und der vierten Biegezone ist, wobei der Abstand zwischen der ersten und der zweiten Biegezone größer als der Abstand zwischen der zweiten und der dritten Biegezone ist, und wobei das Verschlusselement (224) in einer vollständig geöffneten Position eine im Allgemeinen rechteckige Auslassöffnung bereitstellt.

7. Entleerbarer Ostomiebeutel (10) nach einem der Ansprüche 1-6, wobei die mindestens eine Stange (36, 38, 40, 42) vier Stangen (36, 38, 40, 42) beinhaltet, wobei jede der vier Stangen auf einer Außenfläche des Körpers (25) nahe der zweiten Öffnung (46) angeordnet ist, so dass jede der vier Biegezonen (28, 30, 32, 34) zwischen benachbarten Stangen angeordnet ist.

8. Entleerbarer Ostomiebeutel (10) nach einem der Ansprüche 1-7, wobei das Verschlusselement (24) ein weiches Material (52) beinhaltet, das nahe der zweiten Öffnung (46) angeordnet ist, wobei das weiche Material (52) dazu konfiguriert und angeordnet ist, zusammengedrückt zu werden, wenn das Verschlusselement (24) mit dem Schieber (26) in Eingriff steht, um einen flüssigkeitsdichten Dichtungsverschluss bereitzustellen.

9. Entleerbarer Ostomiebeutel (10) nach Anspruch 8, wobei das weiche Material (52) an inneren Oberflächen des Körpers (25) gegenüber der mindestens einen Stange (36, 38, 40, 42) angebracht ist.

10. Entleerbarer Ostomiebeutel (10) nach Anspruch 8, wobei das weiche Material (52) auf äußeren Oberflächen des Körpers (25) zwischen dem mindestens einem Stab (36, 38, 40, 42) und dem Körper (25) angeordnet ist.

11. Entleerbarer Ostomiebeutel (10) nach einem der Ansprüche 8-10, wobei das weiche Material (52) aus einem Schaummaterial gebildet ist.

12. Entleerbarer Ostomiebeutel (10) nach einem der Ansprüche 1-11, wobei das Verschlusselement (24) in dem Auslassabschnitt (18) angeordnet ist, so dass sich die mindestens eine Stange (36, 38, 40, 42) über die Auslassöffnung hinaus erstreckt, wobei äußere Oberflächen des Körpers (25) an benachbarten inneren Oberflächen des Auslassabschnitts (18) angebracht sind, wobei die Auslassvorrichtung (22) dazu konfiguriert und angeordnet ist, um es einem Benutzer zu ermöglichen, entlang Seiten des Auslassabschnitts (18) zu drücken, um die Auslassöffnung bereitzustellen, welche die vier abgewinkelten Ecken aufweist.

13. Entleerbarer Ostomiebeutel (10) nach einem der Ansprüche 1-12, wobei der entleerbare Ostomiebeutel ein zweiteiliges Befestigungssystem beinhaltet, das einen ersten und einen zweiten Befestigungsstreifen (58, 60) umfasst, die zum Halten des Auslassabschnitts (18) in einer zusammengefalteten geschlossenen Position konfiguriert sind.

14. Entleerbarer Ostomiebeutel (10) nach Anspruch 13, wobei der Auslassabschnitt (18) dazu konfiguriert ist, zweimal zusammengefaltet zu werden, um den ersten und den zweiten Befestigungsstreifen (58, 60) in Eingriff zu bringen, um den gefalteten Auslassabschnitt (18) zu halten, wobei der Auslassabschnitt (18) dazu konfiguriert ist, sich entlang eines oberen Umfangs (62) des Verschlusselements nahe der ersten Öffnung (44) für eine erste Faltung zu falten und entlang eines unteren Umfangs (64) des Verschlusselements nahe der zweiten Öffnung (46) für die zweite Faltung zu falten.

15. Entleerbarer Ostomiebeutel (10) nach einem der Ansprüche 1-14, wobei der Schieber (26) einen im Allgemein U-förmigen Körper aufweist, der einen Kanal (54) beinhaltet, der dazu konfiguriert ist, einen Umfangsabschnitt des Verschlusselements (24) nahe der zweiten Öffnung, welche die mindestens eine Stange (36, 38, 40, 42) beinhaltet, aufzunehmen, wobei die Auslassvorrichtung (22) dazu konfiguriert ist, eine flüssigkeitsdichte Abdichtung bereitzustellen, wenn der Schieber (26) mit dem Verschlusselement (24) in Eingriff steht und Druck über dem Umfangsabschnitt des Verschlusselements ausübt.

16. Entleerbarer Ostomiebeutel (10) nach einem der Ansprüche 1-15, wobei der Schieber (26) dazu konfiguriert ist, in Längsrichtung zusammenzufalten und in einer offenen Position über einen Seitenumfang des Auslassabschnitts (18) zu gleiten.

## Revendications

1. Poche de stomie drainable (10), comprenant :
une paroi côté corps (14) et une paroi extérieure (12) définissant une cavité entre elles pour recueillir l'évacuation de stomie ;
une partie d'orifice de sortie (18) s'étendant vers le bas se terminant par une ouverture d'orifice de sortie (20) pour drainer le contenu d'évacuation de stomie recueilli dans la cavité ; et
un dispositif d'orifice de sortie (22) fixé à la partie d'orifice de sortie (18), dans laquelle le dispositif d'orifice de sortie (22) comprend un élément de fermeture (24) et un curseur (26), **caractérisée en ce que** l'élément de fermeture (24) comprend :
un corps (25) comportant une première ouverture (44), une seconde ouverture (46) et quatre zones de courbure (28, 30, 32, 34) définies entre elles ; et
au moins une barre (36, 38, 40, 42) agencée sur le corps à proximité de la seconde ouverture (46) ;
dans laquelle l'élément de fermeture (24) est conçu pour se plier le long des quatre zones de courbure (28, 30, 32, 34), pour fournir une ouverture d'évacuation (44, 46) ayant quatre coins inclinés dans une position ouverte, et pour être pressé à plat dans une position fermée ;
dans laquelle le curseur (26) est conçu pour coulisser sur l'au moins une barre (36, 38, 40, 42) et pour venir en prise avec l'élément de fermeture (24) afin de fermer l'ouverture d'évacuation.

2. Poche de stomie drainable (10) selon la revendication 1, dans laquelle les quatre zones de courbure (28, 30, 32, 34) s'étendent longitudinalement le long du corps entre les première et seconde ouvertures (44, 46).

3. Poche de stomie drainable (10) selon la revendication 1 ou 2, dans laquelle le corps (25) est constitué, d'un seul tenant, d'un matériau polymère, dans laquelle les zones de courbure (28, 30, 32, 34) sont définies par des lignes de pliage ou des lignes perforées.

4. Poche de stomie drainable (10) selon la revendication 1 ou 2, dans laquelle le corps (25) est constitué de quatre pièces d'un matériau polymère en feuille, dans laquelle chaque pièce définit un panneau du corps (25), dans laquelle quatre limites entre les panneaux définissent les quatre zones de courbure (28, 30, 32, 34).

5. Poche de stomie drainable selon l'une quelconque des revendications 1 à 4, dans laquelle les zones de courbure (28, 30, 32, 34) sont agencées symétriquement, dans laquelle les distances entre des zones de courbure adjacentes sont généralement égales les unes aux autres, et dans laquelle l'élément de fermeture (24) fournit une ouverture d'évacuation de forme généralement carrée dans une position complètement ouverte.

6. Poche de stomie drainable (10) selon l'une quelconque des revendications 1 à 4, dans laquelle les zones de courbure (228, 230, 232, 234) comportent des première (228), deuxième (230), troisième (232) et quatrième (234) zones de courbure, dans laquelle la première zone de courbure est adjacente aux deuxième et quatrième zones de courbure, dans laquelle une distance entre les première et deuxième zones de courbure est généralement égale à une distance entre les troisième et quatrième zones de courbure, et une distance entre les deuxième et troisième zones de courbure est généralement égale à une distance entre les première et quatrième zones de courbure, dans laquelle la distance entre les première et deuxième zones de courbure est supérieure à la distance entre les deuxième et troisième zones de courbure, et dans laquelle l'élément de fermeture (224) fournit une ouverture d'évacuation généralement rectangulaire dans une position complètement ouverte.

7. Poche de stomie drainable (10) selon l'une quelconque des revendications 1 à 6, dans laquelle l'au moins une barre (36, 38, 40, 42) comporte quatre barres (36, 38, 40, 42), dans laquelle chacune des quatre barres est agencée sur une surface extérieure du corps (25) à proximité de la seconde ouverture (46), de sorte que chacune des quatre zones de courbure (28, 30, 32, 34) est agencée entre des barres adjacentes.

8. Poche de stomie drainable (10) selon l'une quelconque des revendications 1 à 7, dans laquelle l'élément de fermeture (24) comporte un matériau souple (52) agencé à proximité de la seconde ouverture (46), dans laquelle le matériau souple (52) est conçu et agencé pour être comprimé lorsque l'élément de fermeture (24) vient en prise avec le curseur (26) pour fournir une fermeture étanche aux liquides.

9. Poche de stomie drainable (10) selon la revendication 8, dans laquelle le matériau souple (52) est fixé à des surfaces intérieures du corps (25) faisant face à l'au moins une barre (36, 38, 40, 42).

10. Poche de stomie drainable (10) selon la revendication 8, dans laquelle le matériau souple (52) est agencé sur des surfaces extérieures du corps (25) entre l'au moins une barre (36, 38, 40, 42) et le corps (25).

11. Poche de stomie drainable (10) selon l'une quelconque des revendications 8 à 10, dans laquelle le matériau souple (52) est constitué d'un matériau en mousse.

12. Poche de stomie drainable (10) selon l'une quelconque des revendications 1 à 11, dans laquelle l'élément de fermeture (24) est agencé dans la partie d'orifice de sortie (18), de sorte que l'au moins une barre (36, 38, 40, 42) s'étend au-delà de l'ouverture d'orifice de sortie, dans laquelle des surfaces extérieures du corps (25) sont fixées à des surfaces intérieures adjacentes de la partie d'orifice de sortie (18), dans laquelle le dispositif d'orifice de sortie (22) est conçu et agencé pour permettre à un utilisateur d'appuyer le long des côtés de la partie d'orifice de sortie (18) pour fournir l'ouverture d'évacuation ayant les quatre coins inclinés.

13. Poche de stomie drainable (10) selon l'une quelconque des revendications 1 à 12, dans laquelle la poche de stomie drainable comporte un système de fixation en deux parties comprenant des première et seconde bandes de fixation (58, 60) conçues pour fixer la partie d'orifice de sortie (18) dans une position fermée repliée.

14. Poche de stomie drainable (10) selon la revendication 13, dans laquelle la partie d'orifice de sortie (18) est conçue pour être repliée deux fois pour venir en prise avec les première et seconde bandes de fixation (58, 60) afin de fixer la partie d'orifice de sortie pliée (18), dans laquelle la partie d'orifice de sortie (18) est conçue pour se plier le long d'une périphérie supérieure (62) de l'élément de fermeture à proximité de la première ouverture (44) pour un premier pliage et un pliage le long d'une périphérie inférieure (64) de l'élément de fermeture à proximité de la seconde ouverture (46) pour le second pliage.

15. Poche de stomie drainable (10) selon l'une quelconque des revendications 1 à 14, dans laquelle le curseur (26) a un corps généralement en forme de U comportant un canal (54) conçu pour recevoir une partie périphérique de l'élément de fermeture (24) à proximité de la seconde ouverture comportant l'au moins une barre (36, 38, 40, 42), dans laquelle le dispositif d'orifice de sortie (22) est conçu pour fournir un joint étanche aux liquides lorsque le curseur (26) vient en prise avec l'élément de fermeture (24) et applique une pression sur la partie périphérique de l'élément de fermeture.

16. Poche de stomie drainable (10) selon l'une quelconque des revendications 1 à 15, dans laquelle le curseur (26) est conçu pour se replier longitudinalement et coulisser sur une périphérie latérale de la partie d'orifice de sortie (18) dans une position ouverte.
